# EUROPEAN PATENT SPECIFICATION

(11) **EP 2 437 816 B1**
(45) Date of publication and mention of the grant of the patent: **24.04.2013**
(21) Application number: 10724773.6
(22) Date of filing: 04.06.2010
(51) Int. Cl.: A61M 5/28, A61M 5/315

(54) **MIXING DEVICE WITH PISTON COUPLING ARRANGEMENT**
MISCHVORRICHTUNG MIT KOLBEN-/KOLBENSTANGENBLOCKIERUNG
Dispositif de mélange avec un piston/inter-verrouillage de tige de piston

(30) Priority: 04.06.2009 EP 09161926; 09.06.2009 US 185377 P
(43) Date of publication of application: 11.04.2012
(73) Proprietor: Novo Nordisk Health Care AG, 8050 Zürich (CH)
(72) Inventor: BENDIX, Klaus, DK-2880 Bagsværd (DK); REISENHUS, Michael, Bech, DK-2880 Bagsværd (DK); LJUNGGREEN, Henrik, DK-2880 Bagsværd (DK)
(74) Representative: Vejgaard-Nielsen, Jeppe
(86) International application number: PCT/EP2010/057849
(87) International publication number: WO 2010/139793

(56) References cited:
- DE-A1- 2 450 263
- DE-A1-102005 038 497
- JP-U- 2 058 446
- US-A- 3 380 451
- US-A- 5 643 218
- US-A1- 2006 178 638

## Description

### FIELD OF THE INVENTION

The present invention relates to a device for mixing substances, e.g. for mixing two liquids or for mixing a liquid and a powder, to obtain an injectable medical product. The device is suitable for use regardless of whether the medical product is intended for subcutaneous, intramuscular or intravenous administration.

### BACKGROUND OF THE INVENTION

It is sometimes preferred to keep individual constituents of a medical product separated until just prior to administration. This is for example the case when the medical product in the administrable form has an unacceptably short shelf life. Traditionally, a mixing of two constituents is performed using a syringe with a needle to withdraw the one constituent from one vial and inject it into another vial containing the other constituent, whereupon the latter vial may be shaken to thoroughly mix the two constituents. The syringe with the attached needle is then used to withdraw from this vial the desired amount of drug to be injected into the patient. This kind of manual operation may bring about some uncertainty regarding the exact concentration of the resulting drug, because it can be difficult to completely empty the vials. Moreover, since the first constituent is withdrawn from one vial and transported to another vial via a syringe with a needle, both sterility and safety may be compromised.

Dedicated mixing devices exist which provide for a semi-automated mixing procedure that does not involve moving a needle between separate vials. These devices are typically prefilled with specific volumes of the constituents to be mixed, and the constituents are arranged separately until the user starts using the device. At this point by one or more relatively simple operations the user is able to mix the constituents in the device without having to worry about obtaining the right concentration of the end product.

US 3,380,451 and US 4,479,578 disclose different examples of so-called dual chamber mixing devices in which a diluent is transported from a first chamber through, respectively around, a separating piston to a second chamber to mix with a powder medicament.

WO 2006/058435 discloses a dual chamber cartridge, wherein the transport of a constituent from a rear chamber to a front chamber, holding another constituent, is enabled by a bypass channel in the cartridge wall. The two constituents are originally separated by a central piston having a recess. When it is desired to mix the constituents an actuating piston is moved towards the central piston by means of a piston rod, whereby the constituent in the first chamber is transported to the second chamber through the bypass channel. The actuating piston is provided with a projection which is adapted to mate with the recess of the central piston so as to interlock the two pistons following a complete emptying of the first chamber. The two pistons are formed of similar material.

When administering a medical product intravenously it is important to assure that a vein has been properly punctured before commencing an injection or infusion. Otherwise, the medical product will be delivered to the surrounding tissues and the effect will be lowered or completely lost. It is therefore desirable that mixing devices which are also to be used for intravenous administration are capable of performing a check of whether the delivery element, e.g. a needle, is actually positioned in a vein.

To ascertain a correct needle placement when administering a medical product e.g. subcutaneously is in fact also important. In such cases it is desirable to be able to assure that the needle is not positioned in a blood vessel before an injection is made.

### SUMMARY OF THE INVENTION

In the case of dual chamber devices one way of performing such a check is to retract the most distally positioned piston (the central or front piston) in the device a small distance following the needle insertion to thereby create a negative pressure in the medicament chamber. This has the effect that a small volume of body fluid in the immediate vicinity of the introduced needle is aspirated into the device. If the needle is inserted into a vein blood will enter the device, and the user can therefore easily verify a correct placement of the needle.

To be able to controllably retract the front piston in such a device it is, however, necessary to establish a stable coupling between an operable element and the front piston. The inventors have discovered that providing a projection on a conventional rubber piston and mating it with a recess in a front piston also made of rubber leads to an unstable coupling, due to the inherent flexibility of the rubber-rubber interface, and thereby to an unpredictable, or even no, retraction of the front piston.

It is an object of the invention to provide a drug mixing and delivery device which can be used for subcutaneous, intramuscular and/or intravenous administration and which ensures a proper control of the aspiration procedure allowing a user to check whether a delivery needle has been inserted correctly.

It is another object of the invention to provide such a device, which is simple, safe and easy to use, and for which a minimum of effort is required to mix the contents.

In the disclosure of the present invention, aspects and embodiments will be described which will address one or more of the above objects and/or which will address objects apparent from the below disclosure as well as from the description of exemplary embodiments.

In a first aspect of the invention a drug delivery device as defined by Claim 1 is provided.

Thereby, a device for mixing substances and delivering a final product is provided, the device comprising:
- a container having an outlet,
- a first movable structure arranged in the container to define a first variable volume chamber therein,
- a second movable structure arranged in the container to define a second variable volume chamber therein,
- first and second substances, respectively arranged in the first and second variable volume chambers,
- a third movable structure being operable to move the second movable structure,
- passage means adapted to enable fluid communication between the first variable volume chamber and the second variable volume chamber, and
- coupling means for irreversibly coupling the first movable structure and the second movable structure so as to ensure common subsequent motion of said two structures relative to the container.

In the present context it is understood that the term "irreversibly" refers to normal situations of use of the device.

The container may comprise a body, a first end and a second end, and the coupling means may function to provide for coupling between the first movable structure and the third movable structure such that the third movable structure is operable to move the first movable structure both towards the first end and towards the second end. Further, the first end may be closed by a penetrable wall.

Specifically, the container may be a cartridge suitable for storage of the individual substances as well as for storage and delivery of the mixed product. The first movable structure may serve to separate the individual substances before mixing and may be a first, or front, piston arranged sealingly in the cartridge in such a manner that it is able to slide axially in response to forces being applied to it. The second movable structure may initially serve to separate the second substance from the surroundings and may be a second, or rear, piston arranged sealingly in the cartridge in such a manner that it is able to slide axially in response to forces being applied to it. The cartridge may be made of e.g. glass or plastic and may comprise a generally tubular body which is open at one end and closed at the other end by a penetrable septum. Such a cartridge is typically provided with a mechanical interface which allows for connection with means, e.g. a hypodermic needle or an infusion set, suitable for conveying an injectable product to the body of a person.

The first and second movable structures may be made of identical or different materials. One or both of the movable structures may e.g. be made of an elastomeric material. Alternatively, one or both of the movable structures may comprise an elastomeric material, at least associated with the portions of the surfaces which interact with the container body. When in the following reference is made to a material of the respective first and second movable structures it is understood that the movable structure in question may either completely or partially comprise this material.

The third movable structure may comprise an elongated rod or an operationally similar element being mechanically coupled with the second movable structure, at least during use of the device.

The passage means may comprise a bypass channel, e.g. one or more structural irregularities in the container body or associated with the periphery of the first movable structure, allowing the second substance to move round the first movable structure when the second movable structure is moved towards the first end. Additionally, or alternatively, the passage means may comprise a through-going channel in the first movable structure enabling flow of the second substance through the first movable structure when the second movable structure is moved towards the first end. The through-going channel may be opened automatically for fluid transfer from the second variable volume chamber to the first variable volume chamber when the second movable structure is moved towards the first end, e.g. via a pressure activated valve.

The coupling means may comprise a projecting member or element adapted to couple with suitable means, e.g. a recess, in or on the first movable structure in such a manner that when coupled the third movable structure is operable to selectively position the first movable structure in the cartridge, i.e. to move the first movable structure towards the second end as well as towards the first end. This may be accomplished by a first contact surface on the projecting member cooperating with a second contact surface in e.g. the recess to provide a harpoon-like gripping interface between the second or third movable structure and the first movable structure. Alternatively, or additionally, the coupling means may comprise a recess adapted to couple with a projecting member associated with the first movable structure.

The projecting member and/or the recess comprises, e.g. at least at the surface thereof, a material having a modulus of rigidity, or shear modulus, which is larger than the modulus of rigidity of the first and/or second movable structure. This provides for a stiffer coupling arrangement than a coupling between two elastomers, and thereby ensures both an easier attachment of the projecting member to the recess, since the projecting member does not tend to bend due to its larger resistance to transverse displacement, and a firmer hold between the two once the projecting member is received in the recess. It is noted that if both the projecting member and the recess comprises a material having a larger modulus of rigidity than that/those of the first and/or second movable structure, the material need not be the same (i.e. the projecting member may comprise a material having one modulus of rigidity, and the recess may comprise a material having another modulus of rigidity).

Specifically, the projecting member and/or the recess may comprise a plastic material, a metal, a ceramic, and/or glass.

In one embodiment, the first movable structure, or the first piston, is a rubber piston of the type conventionally used in the art of medical injection/infusion devices, being provided with a recess in the proximally oriented surface, i.e. the surface facing the second, or rear, end of the container. The second movable structure, or the second piston, is a rubber piston comprising first engagement means arranged at the proximally oriented surface thereof for coupling to one element and second engagement means arranged at the distally oriented surface thereof for coupling to another element. The third movable structure is a piston rod comprising at the distal end portion thereof means for coupling to the first engagement means. Further, an insert member is provided having means for coupling to the second engagement means and comprising a projecting body portion, at least the surface of the projecting body portion being of plastic. The insert member may thus e.g. be made of plastic or the projecting body portion may be coated with plastic.

Such an arrangement allows for a rigid member to be introduced into the recess, which results in an easier coupling procedure because the projecting body portion will not tend to deflect laterally during the insertion. Furthermore, the plastic surface may be processed so as to decrease the friction between the projecting body portion and the recess material. Thus, a lower axial force is required to couple the two pistons than would be the case if two rubber surfaces interacted during the insertion of the projecting body portion into the recess. The plastic-rubber contact interface additionally results in a more robust grip between the projecting body portion and the recess thereby providing a greater resistance to axial separation.

The respective first and second engagement means may comprise e.g. a screw thread connection, a snap-fit connection, a bayonet connection, or another suitable connection providing for a firm coupling to the piston rod, respectively the insert member.

In one embodiment, the first movable structure, or the first piston, is a rubber piston of the type conventionally used in the art of medical injection/infusion devices, being provided with a recess in the proximally oriented surface, and the second movable structure, or the second piston, is a rubber piston having an axially, e.g. centrally located, through-going hole. The third movable structure is a piston rod made of plastic which comprises, at the distal end portion thereof, a coupling head for coupling with the recess. The piston rod projects through the hole in the second piston and is sealingly interlocked with the second piston, ensuring simultaneous axial movements of the two. Thereby, the piston rod is operable to selectively position the second piston in the cartridge. In that respect, the piston rod may e.g. comprise engagement members capable of interlocking with corresponding engagement members on the piston rod.

Such an arrangement requires only two elements, the piston rod and the second piston, to provide advantages similar to those described in relation to the previous embodiment. Furthermore, since the piston rod itself is being coupled to the first piston an even more rigid coupling arrangement is actually obtained because the elastic properties of the second piston can then not negatively influence the insertion of the coupling head into the recess (e.g. by allowing a lateral deflection of the distal end portion of the piston rod).

The interlock between the piston rod and the second piston may be adjustable prior to assembly of the delivery device. In that case positioning means may be provided for defining an exact position of the piston rod relative to the second piston, at least one positioning means being associated with the piston rod so as to selectively enable and disable axial displacement of the piston rod relative to the second piston. The positioning means may comprise sealing faces adapted to be moved between positions in which they are in fluid tight contact with respective inner and outer surfaces of the second piston and positions in which they are not in contact with the second piston.

The sealing faces may be positioned via selective fastening/unfastening of two elements in a screw thread connection, e.g. a nut member on a threaded portion of the piston rod, the nut member itself being capable of establishing a fluid tight contact with the second piston, or a coupled segmented piston rod having a sealing flange on each segment. Hence, the adjustable interlock may be provided by incorporating means for varying the axial distance between at least two sealing faces associated with e.g. the piston rod.

In such an arrangement the radial dimension of the piston rod may be smaller than the radius of the through-going hole in the second piston. This enables an easy sterilisation of the piston assembly, comprised of the second piston, the piston rod and optionally a nut member, in an aseptic environment prior to assembly of the delivery device, since when the sealing faces are not in contact with e.g. the piston end surfaces a small clearance is provided throughout the piston assembly allowing for easy steam entry and removal. Following the sterilisation process the piston assembly can be quickly tightened in a single machine before leaving the aseptic area.

The sealing faces may comprise dedicated sealing structures, such as circumferential ridges or rails, adapted to provide increased contact pressure areas when contacting the second piston. These increased contact pressure areas will improve the internal sealing of the piston assembly. The ridges may be arranged for contacting the respective proximal and distal ends of the second piston. Alternatively, or additionally, the ridges may be arranged for contacting one or more wall portions in the through-going hole.

In one embodiment, the coupling means comprises a tubular plastic insert adapted to be occupied in the first piston. The plastic insert is press-fitted into a recess in the first piston, but it could equally well be screw-coupled with, moulded with, or otherwise stably connected to the recess. Alternatively, it could be attached to the first piston e.g. by adhesion, or the like, to a plane surface thereof. The insert comprises a mechanical interface in the form of a hollow, and it comprises barb means, in the form of one or more bent sections, to promote a non-reversible coupling between the first and the second piston.

Such an arrangement involves a mechanical interaction between two coupling parts of which at least one is rigid and has a low friction surface. The plastic barb means contributes to a very firm connection between the recess and a received coupling member associated with the second piston.

In one embodiment, the coupling means comprise a plastic insert adapted to be occupied in the first piston and a piston rod comprising a coupling head adapted to be received in the plastic insert.

In one embodiment, the coupling means comprise a plastic insert adapted to be occupied in the first piston and an insert member for coupling to the second piston, the insert member comprising a projecting body portion made of plastic and adapted to be received in the plastic insert.

A coupling between two rigid components, such as between a plastic projection and a plastic insert, is even firmer than a coupling between a plastic component and a rubber component, and the above arrangement therefore provides for an even more reliable retraction of the first piston. Furthermore, a plastic-plastic interface exhibits less friction than a plastic-rubber interface, and a coupling of a plastic projection to a plastic insert therefore requires a smaller axial force than a coupling of e.g. a plastic projection to a rubber recess.

In one embodiment, the piston rod comprises a first part and a second part adapted to be attached to the first part. The first part is coupled with the second piston and arranged so as to project through the through-going hole therein. The first part comprises a sealing face and attachment means for receiving, or being received by, appropriate attachment means on the second part. The attachment means comprises a snap-fit connection, but may alternatively comprise a bayonet connection, a screw thread connection or other suitable means for stably coupling the two piston rod parts.

An arrangement involving a segmented piston rod provides for a drug delivery device which is shorter and easier to handle in the manufacturing process, e.g. during mounting of the second piston in the cartridge, and which allows for the final marketed product to be delivered in a smaller package. The snap-fit connection provides for an easy assembly and use of the delivery device since only linear movements are required to a) attach the initially separate piston rod part to the piston rod part/rear piston assembly in the cartridge, b) advance the rear piston to mix the two substances and couple the rear piston with the front piston, c) prime the delivery element, d) retract the pistons to check for correct placement of the delivery element in the body, and e) advance the pistons to administer the desired dose of drug. Linear operational movements are strongly preferred e.g. if a quick drug administration is required, or if the user suffers from reduced dexterity.

In the case where the cartridge comprises a bypass channel the first piston may originally be positioned distally of the bypass channel, i.e. between the bypass channel and the second end, thereby isolating the first substance from the second substance. An operation of the piston rod and advancement of the second piston towards the first end will cause the first piston to move towards the first end as well, due to the pressure increase in the second variable volume chamber, until the distal most part of the first piston has passed the distal end of the bypass channel. When this happens the first piston will stop its movement and the liquid will be forced to pass round the first piston until the second variable volume chamber has been collapsed and the second piston abuts the first piston. The collapse of the second variable volume chamber is completed at substantially the same time as the projecting member mates with the recess in the first piston to provide the coupling between the first and the second contact surface. Subsequently, the piston rod may be operated to advance the two pistons simultaneously towards the first end as well as to retract the two pistons simultaneously towards the second end.

A controllable movement of the first piston towards the second end is consequently enabled and a proper aspiration can be performed after mixing of the substances and insertion of a delivery element, such as an infusion needle, through the skin. The retraction of the first piston in the cartridge will create a negative pressure in the first variable volume chamber whereby body fluids in immediate proximity of the opening of the indwelling delivery element will be sucked into the cartridge. Thereby the user can make sure that e.g. a vein has actually been punctured and that an intended intravenous administration can commence.

A coupling between a plastic member, or a member of another rigid material, and a rubber piston, or between two distinct rigid materials, e.g. through a harpoon-like gripping interface, is much firmer than a similar coupling between two rubber components. A mixing device as described in the above therefore provides for better control of the retraction of the first piston subsequent to the coupling with the second piston, and consequently for a more reliable aspiration procedure, because the risk of the coupled components decoupling in the course of the action is considerably lowered.

The present device may be adapted for use in a dedicated drug delivery system, such as a pen injector or the like, or it may be used separately as a stand alone mixing and delivery device. It is understood that e.g. a syringe device may be used equally well as a cartridge to obtain the desired mixing of substances.

During mixing, i.e. during collapse of the second variable volume chamber, a positive pressure is exerted on the second substance to transport it to the first variable volume chamber. In a situation where the coupling means comprise a recess and a projecting member adapted to mate with the recess, it may be difficult to press the projecting member into the recess due to the second substance taking up the space therein. Both in order to displace as much of the second substance to the first variable volume chamber as possible and to avoid counteracting pressure build-up in the recess, which may lead to an unacceptably large coupling force, it is desirable that the drug delivery device is capable of essentially emptying the recess. The coupling means may therefore comprise fluid evacuation means.

In the present context the term "fluid evacuation means" should be understood as any means allowing for evacuation of fluid. More specifically, since the drug delivery device may be a device which reconstitutes a powdered drug using a liquid solvent or which mixes two liquids, liquid may impede a coupling between the first and second piston. The fluid evacuation means are adapted to provide for easy evacuation of liquid entrapped between the first and second piston and consequently ensure an easier and more effective coupling of the two pistons.

In one embodiment, the coupling means comprise a coupling member and a recess for receiving the coupling member. The coupling member comprises an at least partly conical head and the fluid evacuation means comprises a fluid passage, e.g. a slot or a track, in a peripheral portion of the at least partly conical head such that when the at least partly conical head is coupled with the recess at least some of the liquid entrapped in the recess will be allowed to pass the coupling member and thereby escape the recess.

An entrance section of the recess is conically shaped, or may otherwise be provided with an opening which is radially smaller that the largest radial dimension of the coupling member, so that the coupling member must be pressed through the opening, thereby momentarily deforming the entrance section, the coupling member, or both, to firmly couple with the first piston.

The coupling head may alternatively comprise e.g. an at least partly cubical head or an at least partly spherical head. Further, the fluid evacuation means may alternatively, or additionally, comprise e.g. a through-going channel in the coupling member or in the first piston, or a groove or track in the first piston.

In one embodiment, the coupling means comprise a coupling member and a recess at least partly occupying an insert being of a material which is, under normal use conditions of the device, more rigid than the material of the first piston. The insert comprises two or more spaced apart bent sections providing a contact interface for coupling with the coupling member. The spaces between the bent sections function as fluid evacuation canals when the coupling member is pressed into the recess.

In one embodiment, both the coupling member and the recess comprise fluid evacuation means.

In relation to reducing the force required to couple the first and second pistons it is further desirable that the coupling member can be pressed through the recess opening without experiencing substantial frictional resistance. The coupling means may therefore comprise a portion which has a radially variable dimension.

In one embodiment, the coupling means comprise a coupling member and a recess for receiving the coupling member. The recess comprises an opening section which is radially smaller than the rest of the recess. A portion of the coupling member, such as two or more spaced apart segments, is adapted to pivot so as to provide for a structure which is capable of alternately increasing and decreasing its radial dimension. Such a structure may be biased such that it constantly tries to take up a certain radial position, e.g. via one or more integrated resilient hinges. This will provide for a self-expanding coupling member which can easily be fitted into the recess. When pressing the projection against the recess opening the radially displaceable portion will be deflected medially due to the collapsing force from the opening section, which is smaller than the initial radial dimension of the radially displaceable portion. When the radially displaceable portion of the coupling member has completely passed the recess opening it will expand to its initial radial dimension due to the bias of the hinges.

Once positioned in the recess a contact surface on the radially displaceable portion will cooperate with a contact surface in the recess in a harpoon-like interface which provides a non-reversible coupling between the first piston and the second piston.

The coupling member, e.g. the piston rod, may be moulded, e.g. by injection moulding, to create one or more integrated hinges, or so-called "living hinges", constituting the radially displaceable portion. By constructing the radially displaceable portion such that it consists of two or more spaced apart segments means are at the same time being provided for evacuating fluid entrapped in the recess during the fluid transfer between the two variable volume chambers. A retraction of the inserted coupling member will cause the segments to pivot outwards enhancing the engagement with the contact surface in the recess.

In one embodiment, the coupling means comprise a coupling member and a recess for receiving the coupling member. The recess comprises an insert member having two or more laterally displaceable sections providing a contact interface for coupling with the coupling member. The laterally displaceable sections allow for easy insertion of the coupling member into the recess because they are adapted to move radially in response to an axial advancement of the coupling member through the recess opening. Each laterally displaceable section is biased towards a position in which it contributes to preventing the coupling member from escaping the recess once it has been attached thereto (e.g. by functioning as hooks in a harpoon-like coupling interface). Such biasing may e.g. be realised via respective spring arrangements. The laterally displaceable sections may comprise laterally deflectable bent sections, snap arms which are capable of being pressed into wall portions of the insert member, or other suitable means.

In one embodiment, both the coupling member and the recess insert comprise means for varying their respective radial dimensions.

In a second aspect of the invention, a method is provided for coupling a first piston and a second piston in a medical device, the first piston comprising a) a first material having a first modulus of rigidity and b) first coupling means, and the second piston comprising a second material having a second modulus of rigidity, the second piston further being associated with second coupling means, at least one of the first coupling means and the second coupling means comprising a material having a modulus of rigidity which is higher than the first and/or second modulus of rigidity, the method comprising:
o moving the second piston towards the first piston until the first coupling means and the second coupling means interlock.

In one embodiment, moving the second piston towards the first piston comprises performing a purely linear motion.

In one embodiment, the method further comprises: attaching a piston rod portion to a mating portion associated with the second piston prior to moving the second piston towards the first piston. The attachment of the piston rod portion to the mating portion may involve only linear motion.

It is noted with respect to the above method that the modulus of rigidity of the first material may be equal to the modulus of rigidity of the second material.

In a third aspect of the invention a piston assembly for use in a drug delivery device according to the first aspect of the invention is provided, comprising:
- the rear piston comprising a body, a first end and a second end, the rear piston having a hole extending through the body along an axis from the first end to the second end,
- the piston rod arranged so as to traverse the rear piston and project from the first end,
- a first contact face for contacting at least a portion of the first end,
- a second contact face for contacting at least a portion of the second end, and
- means for varying the distance between the first contact face and the second contact face.

In one embodiment, the means for varying the distance between the first contact face and the second contact face are adapted to vary the axial distance between the first contact face and the second contact face.

In one embodiment, the means for varying the distance between the first contact face and the second contact face comprises a screw thread connection between the piston rod and an associated element. One of the first and second contact faces may be an integrated portion of the piston rod, e.g. a flange, and the other of the first and second contact faces may be provided on the associated element, which may e.g. be a nut member adapted to move along a thread on the piston rod.

In one embodiment, the means for varying the distance between the first contact face and the second contact face comprises a screw thread connection between a first portion and a second portion of the piston rod. The first contact face may be an integrated portion of the first portion of the piston rod, e.g. a flange, and the second contact face may be an integrated portion of the second portion of the piston rod, e.g. another flange. In that case the first portion of the piston rod may project from the first end of the piston and the second portion of the piston rod may project from the second end of the piston.

Such an arrangement is less restrictive with respect to the geometry and dimension of the second portion of the piston rod. This gives the manufacturer greater freedom in the design of the interface to the operator (user or actuator in device). Furthermore, in relation to the assembly of the piston assembly this solution does not require a nut to be mounted along the piston rod.

In one embodiment, the piston comprises a material having a first modulus of rigidity, and the portion of the piston rod which projects from the first end comprises a material having a second modulus of rigidity, the second modulus of rigidity being higher than the first modulus of rigidity.

In one embodiment, the radial dimension of the piston rod is so adapted that fluid communication between the first end and the second end is enabled when none of the first and second contact faces contact the piston.

One or both of the first and second contact faces may comprise sealing structures, such as beads, ridges or the like, to provide an area, or areas, of concentrated contact pressure for improving the sealing of the piston assembly. Alternatively, or additionally, such sealing structures are provided along the longitudinal portion of the piston rod, and/or on the inner piston wall.

In one embodiment, the piston rod comprises a first part which traverses the piston and projects from the first end, and a separate second part adapted to be coupled to the first part to allow for user operation of the piston rod. The coupling of the two piston rod parts may be performed manually, e.g. by the user, or automatically, e.g. by a device using a spring. The coupling interface may e.g. be of the snap-fit type which allows for a simple linear coupling movement. In that case the first part may comprise a connecting piece for receiving a mating portion of the second part (or vice versa). A certain clearance may be incorporated in this coupling to allow for relative axial displacements between the first part and the second part. This is relevant if the piston assembly is used in a drug delivery device as described above and the second part is locked relative to the cartridge, e.g. by an actuator mechanism in a drug delivery system comprising the present drug delivery device, because the piston assembly then allows for proximal, or rearwards, movement of the rear piston, e.g. as a result of the substance in the second variable volume chamber expanding due to temperature changes.

The interlocking of the various movable structures described in the above can in principle be executed using any appropriate geometrical configurations. Non-exhaustive further examples of such are ball and annular snaps, fishhook shapes, and circular arrangements of snap beams.

In the present specification reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in at least that one aspect or embodiment of the invention, but not necessarily in all aspects or embodiments of the invention. It is emphasized, however, that any combination of features, structures and/or characteristics described in relation to the various aspects and embodiments of the invention is encompassed by the invention unless otherwise indicated herein or clearly contradicted by context.

### BRIEF DESCRIPTION OF THE DRAWINGS

In the following the invention will be further described with references to the drawings, wherein
Fig. 1 shows a cross sectional view of a drug delivery device according to an embodiment of the invention before use,
Fig. 2 shows a cross sectional view of the drug delivery device of Fig. 1 during movement of a substance from a rear chamber to a front chamber,
Fig. 3 shows a cross sectional view of the drug delivery device of Fig. 1 following a collapse of the rear chamber and a complete mixing of the substances,
Fig. 4 shows a cross sectional view of the drug delivery device of Fig. 1 following an aspiration,
Fig. 5 shows a perspective view of a piston rod for use in a drug delivery device according to an embodiment of the invention,
Fig. 6 shows a perspective view of another piston rod for use in a drug delivery device according to an embodiment of the invention,
Fig. 7 shows a cross sectional view of the piston rod of Fig. 6,
Fig. 8 shows a cross sectional perspective view of a piston for use in a drug delivery device according to an embodiment of the invention,
Fig. 9 shows a cross sectional view of a coupling between a piston rod and a piston in a drug delivery device according to an embodiment of the invention, and
Figs. 10-17 show various embodiments of a piston assembly for use in a drug delivery device.

In the figures like structures are mainly identified by like reference numerals.

### DESCRIPTION OF EXEMPLARY EMBODIMENTS

When in the following relative expressions, such as "front" and "rear", are used, these refer to the appended figures and not necessarily to an actual situation of use. The shown figures are schematic representations for which reason the configuration of the different structures as well as their relative dimensions are intended to serve illustrative purposes only.

The present invention is directed to devices for storing substances separately and mixing the substances in a simple and easy way when required to obtain an administrable medical drug. A mixing device according to the invention is suitable for use in situations where the drug is to be injected subcutaneously as well as where the drug is to be given intravenously. The mixing device is adapted to be connected with an appropriate delivery element, such as an injection needle or an infusion set, capable of conveying the drug from the device to a subject.

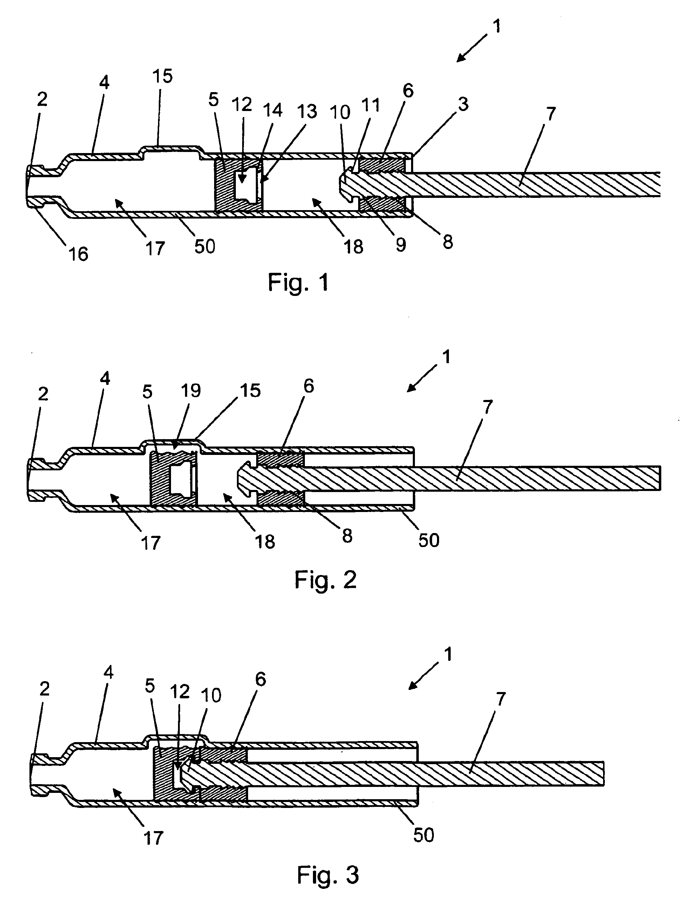

Fig. 1 shows a mixing device 1 according to an embodiment of the invention in a non-use situation. The mixing device 1 comprises a cartridge 50 having a distal end 16 and a proximal end 3. A cartridge wall 4 extends between the distal end 16 and the proximal end 3, constituting a cartridge body. The cartridge body is generally circular-cylindrical and made of glass or transparent plastic. The distal end 16 is closed by a penetrable septum 2 which is adapted to be penetrated by a sharp element (not shown). The proximal end 3 is closed by a rear piston 6 which is in sealing engagement with the inside of the cartridge wall 4 and which is adapted to slide in the cartridge 50 in response to an operation of a piston rod 7.

The piston rod 7 projects through a bore in the rear piston 6 and is non-detachably connected to the rear piston 6 via round-going teeth 8 and an annular flange 9 cooperating with corresponding indentations in the rear piston 6. This connection allows the user to advance the rear piston 6 through the cartridge 50 by pushing the piston rod 7 towards the distal end 16 and to retract the rear piston 6 through the cartridge 50 by pulling the piston rod 7 towards the proximal end 3. The piston rod 7 has a projecting head 10 of conical shape providing an annular contact face 11.

A front piston 5 is slidably positioned in the cartridge 50 between the rear piston 6 and a rear end of a bead 15 in the cartridge wall 4. The front piston 5 has a recess 12 which is adapted to interface with the projecting head 10. The recess 12 has an opening section 13 which tapers towards the distal end 16 and which is radially smaller than the adjacent part of the recess 12 so as to provide an annular contact face 14.

A front chamber 17 is thus established between the penetrable septum 2 and the front piston 5. The front chamber 17 holds a first substance (not shown) being either a dry drug or a liquid. Further, a rear chamber 18 is established between the front piston 5 and the rear piston 6, and the rear chamber 18 holds a liquid (not shown).

Fig. 2 shows the mixing device 1 in a liquid transfer situation where the piston rod 7 has been pushed a distance towards the distal end 16. Movement of the piston rod 7 is accompanied by a movement of the rear piston 6 due to the non-detachable connection between the two. The advancement of the rear piston 6 in the cartridge 50 pressurises the liquid in the rear chamber 18. The increased pressure in the rear chamber 18 will force the front piston 5 to move towards the distal end 16 as well until the rear end of the front piston 5 has passed the rear end of the bead 15. When this happens a bypass channel 19 is established between the bead 15 and the front piston 5 allowing the liquid to pass round the front piston 5 and to enter the front chamber 17, where it is mixed with the first substance.

Fig. 3 shows the mixing device 1 in a situation where the rear chamber 18 has been completely collapsed and the projecting head 10 has mated with the recess 12 to provide a non-reversible coupling between the piston rod 7 and the front piston 5. The transferable amount of liquid has now been moved to the front chamber 17 and mixed with the first substance to produce, or restore, an injectable medical product.

In case the medical product is intended for e.g. subcutaneous or intramuscular administration the mixing device 1 is in principle ready to use. The user then simply connects a needle (not shown) to the distal end 16, e.g. by connecting a needle hub to a suitable interface on a holder (not shown) for the cartridge 50, inserts the needle through the skin, and begins to push the piston rod 7 further towards the distal end 16, whereby both the front piston 5 and the rear piston 6, being in abutting engagement, will advance through the cartridge 50 to expel the medical product from the front chamber 17 through the needle and into the desired compartment.

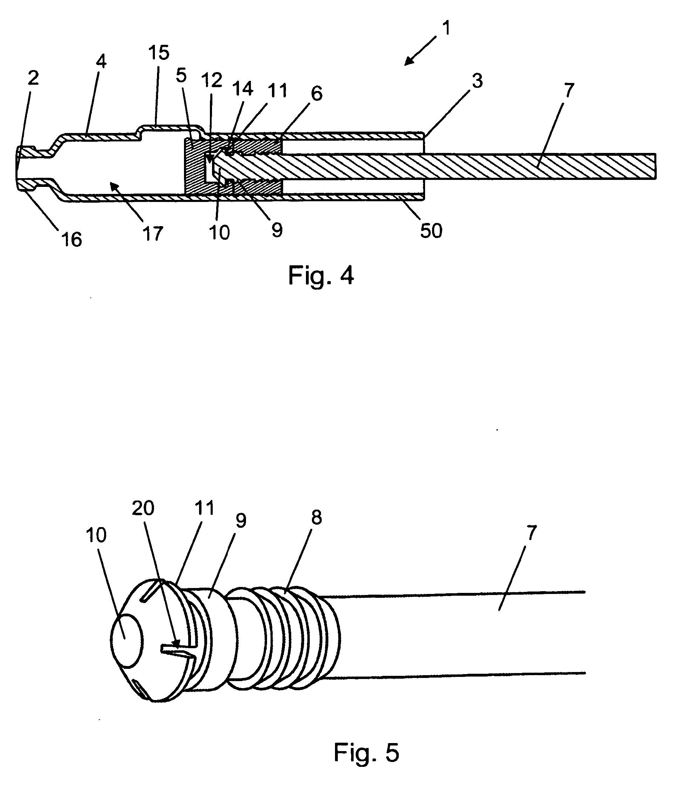

In case the medical product is intended for intravenous administration the user connects an IV infusion set to the distal end 16, e.g. in a similar manner as described above, inserts the infusion set needle into the skin and tests whether the needle has been inserted properly in a vein by pulling the piston rod 7 a small distance towards the proximal end 3. When pulling the piston rod 7 towards the proximal end 3 the annular contact face 11 of the projecting head 10 engages with the annular contact face 14 of the recess 12 and the front piston 5 is thereby forced to follow the piston rod 7, whereby a negative pressure differential is created in the front chamber 17. Fig. 4 shows the mixing device 1 in such a situation (the infusion set is not shown).

The negative pressure differential in the front chamber 17 will cause body fluid in the immediate vicinity of the needle to be sucked into the front chamber 17. If blood is thereby sucked into the cartridge 50 the user can conclude that the needle has been positioned in a vein and he can then go on and push the piston rod 7 towards the distal end 16 to inject, or infuse, the medical product. If blood does not show in the cartridge 50 the needle is not positioned correctly and the user can then try to find another location for the needle.

Fig. 5 shows an embodiment of a piston rod 7' for use in the mixing device 1. The piston rod 7 has round-going teeth 8 and an annular flange 9 which are adapted to cooperate with corresponding structures in a rear piston 6, as described in the above in relation to Fig. 1. The projecting head 10 has a number of axially extending slots 20 distributed along its periphery providing for canals through which liquid in the recess 12 can flow to escape the recess 12 when the projecting head 10 is pushed through the opening section 13.

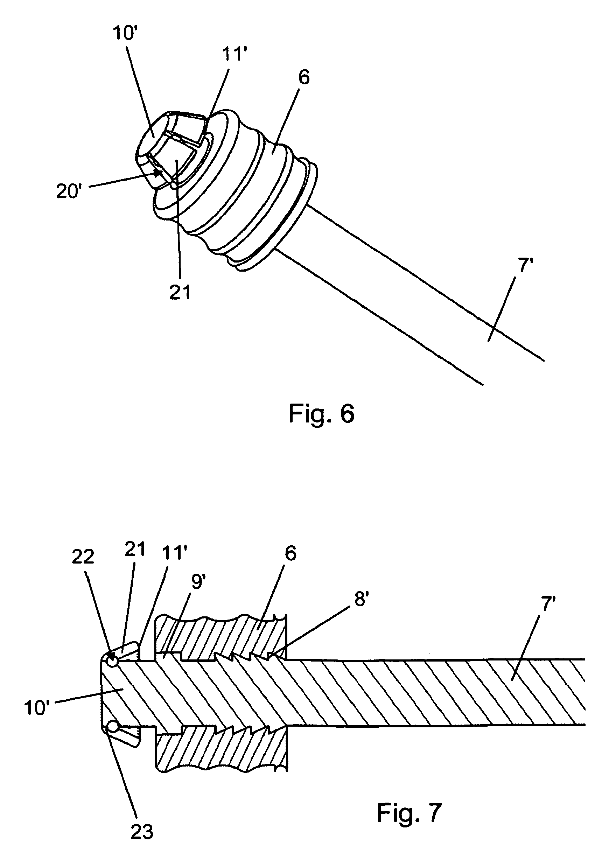

Figs. 6 and 7 show different views of another embodiment of a piston rod 7', including a rear piston 6 attached thereto via locking teeth 8' and a flange 9', for use in the mixing device 1. The projecting head 10' is provided with a number of circumferentially spaced apart segments 21 attached to the piston rod 7' only by thin films of material 23 in a manner similar to so-called "living hinges". In an unloaded state the segments 21 protrude radially from the projecting head 10'. An area 22 of removed material allows each segment 21 to pivot and be pressed against the projecting head 10' when subjected to a radial force, and to return to its initial position once the radial loading ceases. When the projecting head 10' is pushed against the front piston 5 the tapering opening section 13 will force the segments 21 to fold about the projecting head 10' until the segments 21 are completely though the opening section 13. At that point the segments 21 will return to their respective initial positions and a contact face 11' on each segment 21 will provide a harpoon-like interface with the annular contact face 14 to thereby ensure a non-reversible coupling between the piston rod 7' and the front piston 5. As the segments 21 are circumferentially spaced apart a number of passageways 20' are provided through which liquid in the recess 12 can flow to escape the recess 12 when the projecting head 10' is pushed through the opening section 13.

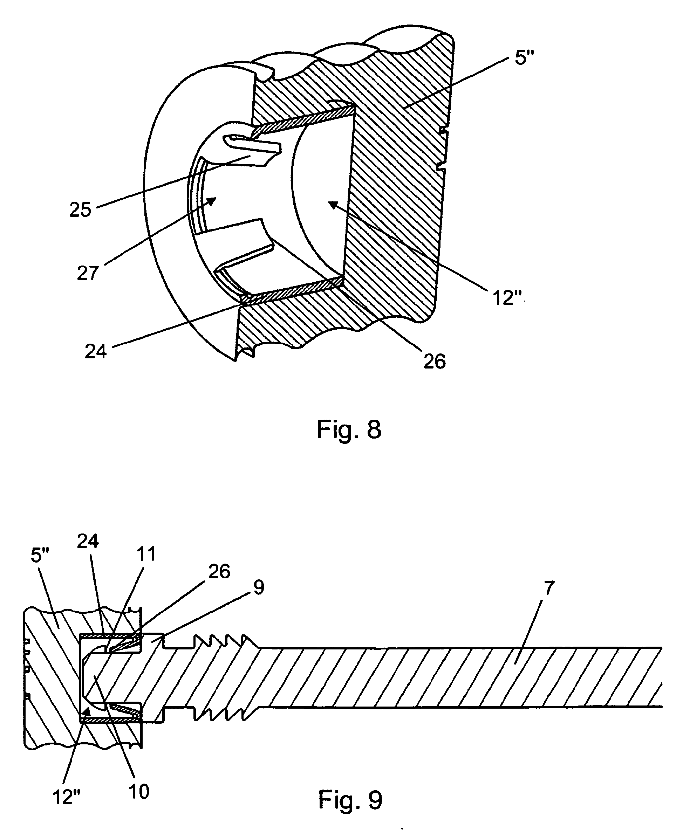

Fig. 8 shows an embodiment of an elastomeric front piston 5" for use in the mixing device 1. The front piston 5" has a recess 12" in which a rigid insert 24 is positioned. The rigid insert 24 is made of plastic, e.g. polypropylene, and is press-fitted into the recess 12" so as to provide a rigid interface for coupling with a piston rod. The rigid insert 24 is segmented and bent in the region of the recess opening to form a collar of circumferentially spaced apart rigid flanges 25 which are able to deflect radially to allow a wider element to pass. Each rigid flange 25 terminates in a contact face 26. As the rigid flanges 25 are circumferentially spaced apart a number of canals 27 are provided allowing for evacuation of liquid from the recess 12" during insertion of a coupling head thereinto. The press-fit connection between the rigid insert 24 and the recess 12" is sufficient to resist pull forces which the rigid insert 24 may experience under normal use of the mixing device 1, and the rigid insert 24 can therefore be considered to be immovably positioned in the front piston 5". Other ways of coupling the rigid insert 24 to the recess 12", e.g. via a screw thread or glue, can alternatively be employed to further secure a durable connection.

Fig. 9 shows the front piston 5" coupled to the piston rod 7 (the rear piston is omitted from this figure for the sake of clarity). The projecting head 10 has been pushed into the recess 12" past the rigid flanges 25 which have deflected inwards towards the front piston 5" during the passage of the radially larger projecting head 10 and which have elastically recovered to their initial positions in which the contact faces 26 will cooperate with the annular contact face 11 when the piston rod 7 is subsequently retracted in the cartridge 50 (not visible). A retraction of the piston rod 7 will thus lead to a firm self-locking coupling between the rigid insert 24 and the projecting head 10. During the insertion of the projection head 10 into the recess 12" the majority of the liquid originally taking up the space in the recess 12" is evacuated therefrom through the canals 27 provided between the rigid flanges 25.

It is clear that the piston rod 7' with the projecting head 10' described in relation to Figs. 6 and 7 could be employed just as well as the piston rod 7 in connection with an embodiment of the mixing device 1 that includes the front piston 5". In that case when the projecting head 10' has been pressed into the recess 12" the contact faces 11' on the hinged segments 21 will cooperate with the contact faces 26 on the rigid flanges 25 in a self-locking stable engagement to ensure a proper and reliable coupling between the piston rod 7' and the front piston 5".

It is also clear that the operations of the piston rod 7, 7' can be performed manually by the user or they can be performed automatically, e.g. by employing the mixing device 1 in a suitable automatic or semi-automatic mixing and delivery device.

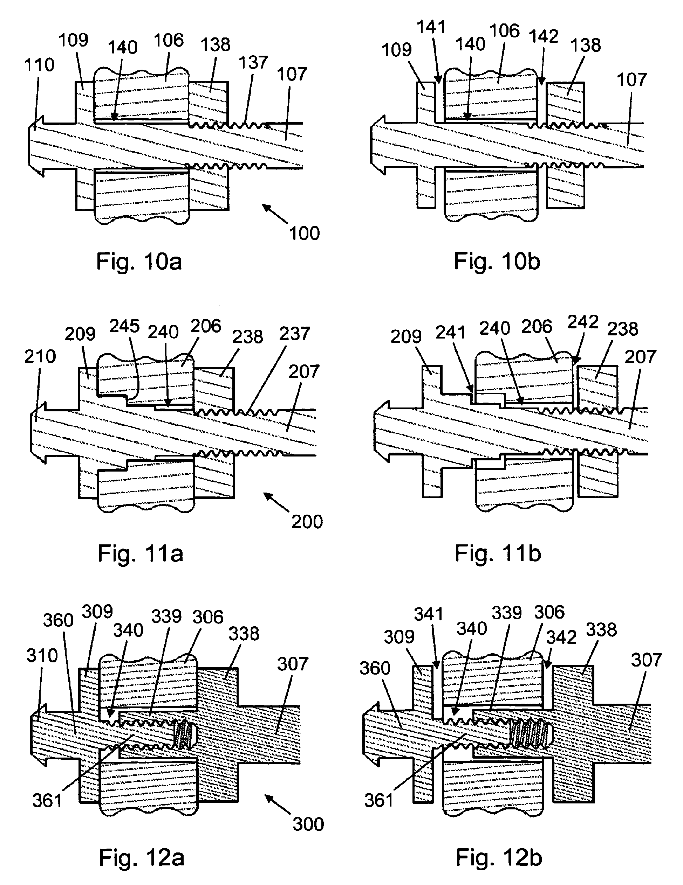

Fig. 10a shows a piston assembly 100 according to an embodiment of the invention. The piston assembly 100 comprises a rubber piston 106 through which a bore 140 extends, a piston rod 107 of plastic arranged so as to traverse the piston 106 through the bore 140, and a nut 138. The front portion of the piston rod 107 is formed to provide a coupling head 110 which is adapted to cooperate with mating coupling means on another piston (not shown). At a certain axial distance from the coupling head 110 a flange 109 is provided. In this figure the flange 109 is in abutment with a front end face of the piston 106. The piston rod 107 further comprises a threaded section 137 along which the nut 138 can be screwed. In the figure the nut 138 is positioned in abutment with a rear end face of the piston 106, thereby providing a fluid tight piston assembly 100.

Fig. 10b shows the piston assembly 100 in a loosened state, where neither the flange 109 nor the nut 138 abuts the piston 106. In this state a clearance 141 is provided between the flange 109 and the front end face of the piston 106, and another clearance 142 is provided between the nut 138 and the rear end face of the piston 106. The radial dimension of the piston rod 107 is chosen so that fluid communication is enabled between the two ends of the piston 106. The piston assembly 100 can therefore be easily sterilised, using e.g. conventional heat sterilisation, such as autoclaving, or chemical sterilisation, because gas is allowed to reach all surfaces of the individual components.

Following the sterilisation the piston assembly 100 can be easily tightened by screwing the nut 138 along the threaded section 137 until both the flange 109 and the nut 138 abut the piston 106, as shown in Fig. 10a. Such tightening can even be performed in the aseptic environment.

In the embodiment shown in Figs. 10a and 10b the piston assembly can be tightened, respectively loosened, by screwing a nut along a thread. However, it is understood that alternative ways of tightening and loosening such a piston assembly can be employed. For example, the piston rod may be adapted to cooperate with a contact member in a snap-fit or bayonet connection arrangement.

Fig. 11a shows a piston assembly 200 according to another embodiment of the invention. The piston assembly 200 is of the same type as the piston assembly 100 but differs from the latter with respect to the arrangement of the sealing faces. The piston assembly 200 comprises a piston 206 having a bore 240, a piston rod 207 arranged so as to traverse the bore 240, and a nut 238. The piston rod 207 is formed to provide a coupling head 206 and a flange 209, and has a threaded section 237 along which the nut 238 can be screwed. A portion of the piston rod 207 traversing the bore 240 is shaped to provide a step-wise change of the radial dimension thereof. Similarly, a portion of the inner wall of the piston 206 is shaped to provide a step-wise change of the diameter of the bore 240. Thereby, an annular abutment surface 245 on the piston rod 207 is provided for abutment with an inner wall portion of the piston 206.

Fig. 11b shows the piston assembly 200 in a loosened state, wherein a clearance 241 is provided between the piston rod 207 and the front end of the piston 206, and another clearance 242 is provided between the nut 238 and the rear end of the piston 206, allowing for easy sterilisation of the piston assembly 200.

Fig. 12a shows a piston assembly 300 according to yet another embodiment of the invention. The piston assembly 300 comprises a rubber piston 306 through which a bore 340 extends, and a piston rod comprising a front portion 360 and a rear portion 307. The front portion 360 comprises a rearwardly projecting cylinder 361 having an external screw thread interface, and the rear portion 307 comprises a receiving section 339 having an internal screw thread for mating with the external screw thread on the cylinder 361. The front portion 360 further comprises a flange 309 for abutment with a front end face of the piston 306. The rear portion 307 further comprises a flange 338 for abutment with a rear end face of the piston 306.

Fig. 12b shows the piston assembly 300 in a loosened state, wherein a clearance 341 is provided between the flange 309 and the front end of the piston 306, and another clearance 342 is provided between the flange 338 and the rear end of the piston 306, allowing for easy sterilisation of the piston assembly 300. The piston assembly 300 is tightened by inducing a clockwise, or anti-clockwise, relative rotational movement between the front portion 360 and the rear portion 307, whereby the screw thread interface between the cylinder 361 and the receiving section 339 will decrease the axial distance between the two flanges 309, 338.

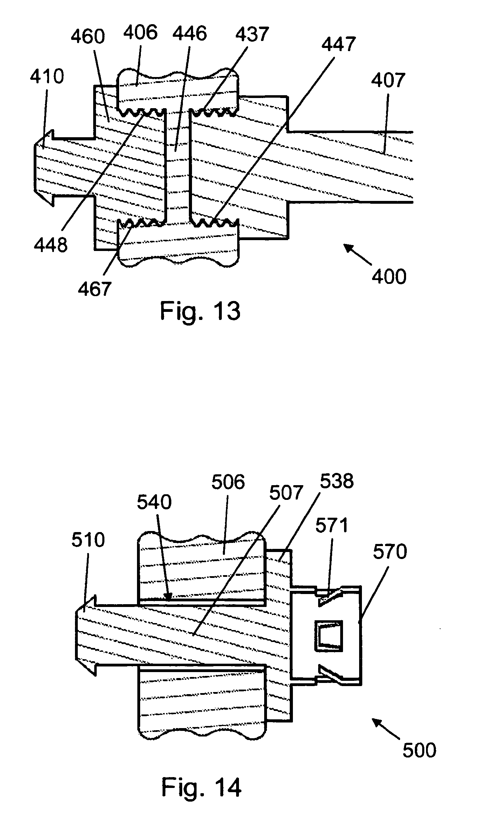

Fig. 13 shows a piston assembly 400 for use in a drug delivery device as described in the above. The piston assembly 400 comprises a rubber piston 406, a piston rod 407, and a projecting member 460. The front end portion of the piston rod 407 has an external screw thread 437 which is in engagement with an internal screw thread 447 of the piston 406. Similarly, the rear end portion of the projecting member 460 has an external screw thread 467 which is in engagement with an internal screw thread 448 of the piston 406. The piston 406 thereby acts as a coupling element between the piston rod 407 and the projecting member 460, the respective front and rear end portions of which being separated by a central wall 446. The projecting member 460 is formed to provide a coupling head 410 which is adapted to interact with a mating recess in another piston (not shown) in a harpoon-like fashion. In this embodiment, no plastic parts need to sustain a specific sealing pressure in order to uphold a fluid tight piston assembly over a period of time, e.g. during storage of the drug delivery device. The only sealing interface is between the periphery of the piston 406 and the glass wall of the drug delivery device container.

Fig. 14 shows a piston assembly 500 comprising a piston 506 having a bore 540, and a piston rod segment 507 piercing through the bore 540. The piston rod segment 507 comprises at its front end a coupling head 510 and at its rear end a connecting piece 570 adapted to receive a mating structure on a second piston rod segment (not shown). A number of snap arms 571 provide a snap-fit coupling interface to the second piston rod segment. The piston rod segment 507 further comprises a flange 538 for abutment with the rear end face of the piston 506 and a coupling interface to a contact member (omitted from the figure) adapted to abut with the front end face of the piston 506, thereby providing a fluid tight piston assembly 500. The contact member may e.g. be adapted for selective placement on the piston rod segment 507 via a screw-coupling, a snap-fit or a bayonet coupling arrangement.

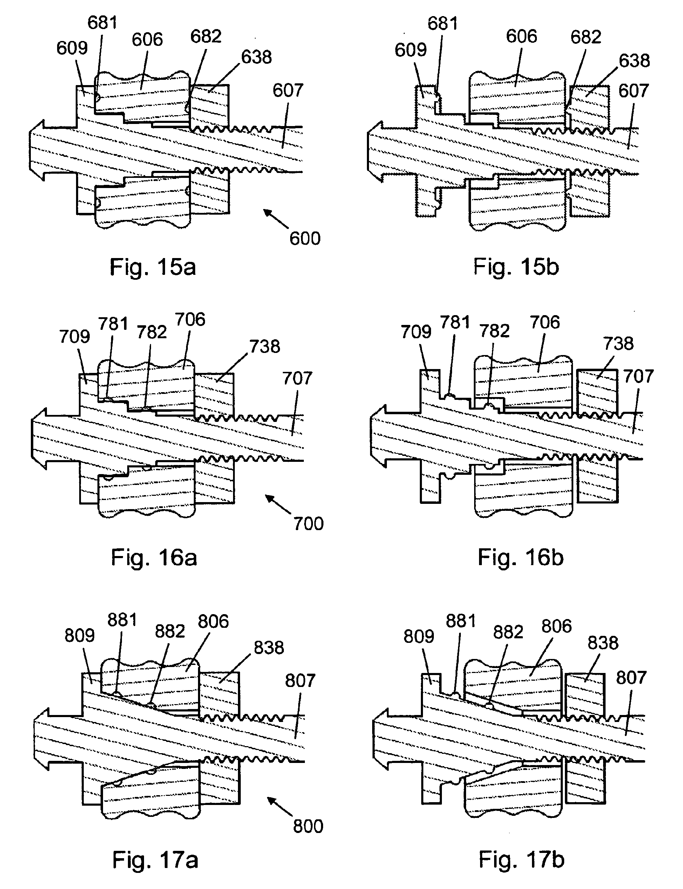

Fig. 15a shows a piston assembly 600 of the type shown in Fig. 11. The piston assembly 600 comprises a piston 606, a piston rod 607, and a nut 638. The piston rod 607 comprises a flange 609 which is adapted to contact the front end face of the piston 606.

The flange 609 is provided with an annular ridge 681 serving as a sealing lip when the flange 609 abuts the piston 606. Similarly, an annular ridge 682 is provided on the nut 638. When the piston assembly 600 is tightened the ridges 681, 682 are pressed tightly against portions on the respective end faces of the piston 606, whereby an improved sealing is obtained.

By arranging the ridges 681, 682 so as to interact with the end faces of the piston 606 the sealing pressure can be managed solely through the tightening of the nut 638. Thereby, the piston assembly 600 is less sensitive to variations of the axial length of the piston 606.

Fig. 15b shows the piston assembly 600 in a loosened state, ready for sterilisation.

Fig. 16a shows a piston assembly 700 of the type shown in Fig. 15. The piston assembly 700 comprises a piston 706, a piston rod 707, and a nut 738. The piston rod 707 comprises a flange 709 which is adapted to contact the front end face of the piston 706. A longitudinal portion of the piston rod 707 is provided with circumferential ridges 781, 782 for sealing contact with the inner wall of the piston 706. In a tightened state of the piston assembly 700 the ridges 781, 782 will provide a fluid tight interface with the inner wall of the piston 706.

By arranging the ridges 781, 782 so as to interact with the inner wall of the piston 706 the plastic-rubber interface will be less stressed because the piston assembly 700 does not have to be tightened to the same degree as if the ridges were arranged on the flange 709 and the nut 738 to interact with the end faces of the piston 706. During storage of a drug delivery device in which the piston assembly 700 is incorporated a long term load effect such as creep of the plastic material is therefore less likely to occur.

Fig. 16b shows the piston assembly 700 in a loosened state, ready for sterilisation.

Fig. 17a shows a piston assembly 800 according to an alternative embodiment of the invention. The piston assembly 800 comprises a piston 806, a piston rod 807, and a nut 838. The piston rod 807 comprises a flange 809 and a conical longitudinal portion tapering rearwards from the flange 809. The conical portion is adapted to be in sealing contact with the inner wall of the piston 806 during use of the piston assembly 800. Two circumferential ridges 881, 882 on the conical portion ensure a fluid tight interface between the piston rod 807 and the piston 806.

Fig. 17b shows the piston assembly 800 in a loosened state, ready for sterilisation.

It is noted that the sealing ridges described with respect to the embodiments shown in Figs. 15-17 can equally well be provided on the piston, or on both the piston rod and the piston.

## Claims

1. A drug delivery device (1) comprising:
- a container (50) comprising a first end (16) and a second end (3),
- a front piston (5, 5") arranged between the first end (16) and the second end (3), defining a first variable volume chamber (17) therebetween,
- a rear piston (6, 106, 206, 306, 406, 506, 606, 706, 806) arranged between the front piston (5, 5") and the second end (3), defining a second variable volume chamber (18) therebetween, the rear piston (6, 106, 206, 306, 406, 506, 606, 706, 806) comprising a material having a first modulus of rigidity,
- a piston rod (7, 7', 107, 207, 307, 407, 507, 607, 707, 807) adapted to move the rear piston (6, 106, 206, 306, 406, 506, 606, 706, 806) with respect to the container (50),
- a first substance arranged in the first variable volume chamber (17),
- a second substance arranged in the second variable volume chamber (18),
- passage means (15) adapted to allow the second substance to move from the second variable volume chamber (18) to the first variable volume chamber (17), and
- coupling means structured to non-releasably couple the front piston (5, 5") and the rear piston (6, 106, 206, 306, 406, 506, 606, 706, 806), the coupling means comprising a material having a second modulus of rigidity,
wherein the second modulus of rigidity is higher than the first modulus of rigidity.

2. A drug delivery device according to claim 1, wherein the coupling means comprise a projecting member (10, 10', 110, 210, 310, 410, 510) and recess means (12, 12") adapted to receive the projecting member (10, 10', 110, 210, 310, 410, 510), and wherein at least a portion of the projecting member (10, 10', 110, 210, 310, 410, 510) or at least a portion of the recess means (12, 12") is of the material having the second modulus of rigidity.

3. A drug delivery device according to claim 2, wherein the projecting member (10, 10', 110, 210, 310, 410, 510) is associated with one of the front piston (5, 5"), the rear piston (6, 106, 206, 306, 406, 506, 606, 706, 806) and the piston rod (7, 7', 107, 207, 307, 407, 507, 607, 707, 807).

4. A drug delivery device according to claim 3, wherein the projecting member (10, 10', 110, 210, 310, 510) is an integrated portion of the piston rod (7, 7', 107, 207, 307, 507, 607, 707, 807).

5. A drug delivery device according to any of claims 2 - 4, wherein the recess means (12") comprises an insert (24), the insert (24) comprising a material having the second modulus of rigidity.

6. A drug delivery device according to any of claims 2 - 5, wherein the projecting member (10, 10', 110, 210, 310, 410, 510) or the recess means (12, 12") comprises fluid evacuation means allowing fluid to pass the projecting member (10, 10', 110, 210, 310, 410, 510) during connection to the recess means (12, 12").

7. A drug delivery device according to claim 6, wherein the projecting member (10, 10') comprises a coupling head, and wherein the fluid evacuation means comprises a slot (20, 20') in the coupling head.

8. A drug delivery device according to any of claims 2 - 7, wherein the projecting member (10, 10', 110, 210, 310, 410, 510) and/or the recess means (12, 12") comprises a displacement portion which is capable of radial displacement.

9. A drug delivery device according to claim 8, wherein the projecting member (10') comprises a coupling head, and wherein the displacement portion comprises a pivotable segment (21) of the coupling head.

10. A drug delivery device according to claim 8 or 9, wherein the recess means (12") comprises an insert (24), and wherein the displacement portion comprises a flange (25) of the insert (24), the flange (25) being radially deflectable so as to enable entry of the projecting member (10, 10', 110, 210, 310, 410, 510) into the recess means (12") and to prevent subsequent escape of the projecting member (10, 10', 110, 210, 310, 410, 510) from the recess means (12").

11. A drug delivery device according to any of the preceding claims, wherein the piston rod comprises a first part (307) and a second part (360) adapted to be coupled to the first part.

12. A piston assembly (100, 200, 300, 600, 700, 800) for use in a drug delivery device according to any of claims 1 - 11, the piston assembly (100, 200, 300, 600, 700, 800) comprising:
- the rear piston (106, 206, 306, 606, 706, 806) comprising a body, a first end and a second end, the rear piston (106, 206, 306, 606, 706, 806) having a hole (140, 240, 340) extending through the body from the first end to the second end,
- the piston rod (107, 207, 307, 607, 707, 807) arranged so as to traverse the rear piston (106, 206, 306, 606, 706, 806) and project from the first end,
- a first contact face for contacting at least a portion of the first end,
- a second contact face for contacting at least a portion of the second end, and
- means for varying the distance between the first contact face and the second contact face.

13. A piston assembly according to claim 12, wherein the means for varying the distance between the first contact face and the second contact face comprises a screw thread connection between the piston rod (107, 207, 607, 707, 807) and an associated element (138, 238, 638, 738, 838).

14. A piston assembly according to claim 12, wherein the means for varying the distance between the first contact face and the second contact face comprises a screw thread connection between a first portion (339) and a second portion (361) of the piston rod (307).

15. A method for coupling a first piston and a second piston in a medical device, the first piston comprising a) a first material having a first modulus of rigidity and b) first coupling means, and the second piston comprising a second material having a second modulus of rigidity, the second piston further being associated with second coupling means, at least one of the first coupling means and the second coupling means comprising a material having a modulus of rigidity which is higher than the first and/or second modulus of rigidity, the method comprising:
○ moving the second piston towards the first piston until the first coupling means and the second coupling means interlock.

## Patentansprüche

1. Arzneimittelabgabevorrichtung (1), umfassend:
- einen Behälter (50), umfassend ein erstes Ende (16) und ein zweites Ende (3),
- einen vorderen Kolben (5, 5"), der zwischen dem ersten Ende (16) und dem zweiten Ende (3) angeordnet ist, wodurch eine erste Kammer mit variablem Volumen (17) dazwischen definiert wird,
- einen hinteren Kolben (6, 106, 206, 306, 406, 506, 606, 706, 806), der zwischen dem vorderen Kolben (5, 5") und dem zweiten Ende (3) angeordnet ist, wodurch eine zweite Kammer mit variablem Volumen (18) dazwischen definiert wird, wobei der hintere Kolben (6, 106, 206, 306, 406, 506, 606, 706, 806) ein Material mit einem ersten Starrheitsmodul umfasst,
- eine Kolbenstange (7, 7', 107, 207, 307, 407, 507, 607, 707, 807), die derart angepasst ist, dass sie den hinteren Kolben (6, 106, 206, 306, 406, 506, 606, 706, 806) in Bezug auf den Behälter (50) bewegt,
- eine erste Substanz, die in der ersten Kammer mit variablem Volumen (17) angeordnet ist,
- eine zweite Substanz, die in der zweiten Kammer mit variablem Volumen (18) angeordnet ist,
- ein Durchgangsmittel (15), das dazu angepasst ist, es der zweiten Substanz zu ermöglichen, sich von der zweiten Kammer mit variablem Volumen (18) zu der ersten Kammer mit variablem Volumen (17) zu bewegen, und
- ein Kupplungsmittel, das derart strukturiert ist, dass es an dem vorderen Kolben (5, 5") und am hinteren Kolben (6, 106, 206, 306, 406, 506, 606, 706, 806) unlösbar gekoppelt ist, wobei das Kupplungsmittel ein Material mit einem zweiten Starrheitsmodul umfasst,
wobei das zweite Starrheitsmodul höher als das erste Starrheitsmodul ist.

2. Arzneimittelabgabevorrichtung nach Anspruch 1, wobei das Kupplungsmittel ein hervorstehendes Element (10, 10', 110, 210, 310, 410, 510) und ein ausgespartes Mittel (12, 12"), das zum Aufnehmen des hervorstehenden Elements (10, 10', 110, 210, 310, 410, 510) des hervorstehendenden Elements (10, 10', 110, 210, 310, 410, 510) angepasst ist, und wobei mindestens ein Teil des hervorstehenden Elements (10, 10', 110, 210, 310, 410, 510) oder mindestens ein Teil des ausgesparten Mittels (12, 12") aus einem Material mit dem zweiten Starrheitsmodul besteht.

3. Arzneimittelabgabevorrichtung nach Anspruch 2, wobei das hervorstehende Element (10, 10', 110, 210, 310, 410, 510) entweder mit dem vorderen Kolben (5, 5"), dem hinteren Kolben (6, 106, 206, 306, 406, 506, 606, 706, 806) oder der Kolbenstange (7, 7', 107, 207, 307, 407, 507, 607, 707, 807) verbunden ist.

4. Arzneimittelabgabevorrichtung nach Anspruch 3, wobei das hervorstehende Element (10, 10', 110, 210, 310, 510) ein integrierter Teil der Kolbenstange (7, 7', 107, 207, 307, 507, 607, 707, 807) ist.

5. Arzneimittelabgabevorrichtung nach einem der Ansprüche 2-4, wobei das ausgesparte Mittel (12") einen Einsatz (24) umfasst, wobei der Einsatz (24) ein Material mit dem zweiten Starrheitsmodul umfasst.

6. Arzneimittelabgabevorrichtung nach einem der Ansprüche 2-5, wobei das hervorstehende Element (10, 10', 110, 210, 310, 410, 510) oder das ausgesparte Mittel (12, 12") ein Fluidentleerungsmittel umfasst, das es ermöglicht, dass Fluid während der Verbindung mit dem ausgesparten Mittel (12, 12") zu dem hervorstehenden Element (10, 10', 110, 210, 310, 410, 510) läuft.

7. Arzneimittelabgabevorrichtung nach Anspruch 6, wobei das hervorstehende Element (10, 10') einen Kupplungskopf umfasst und wobei das Fluidentleerungsmittel einen Schlitz (20, 20') im Kupplungskopf umfasst

8. Arzneimittelabgabevorrichtung nach einem der Ansprüche 2-7, wobei das hervorstehende Element (10, 10', 110, 210, 310, 410, 510) und/oder das ausgesparte Mittel (12, 12") einen Verschiebungsteil umfasst, der zur radialen Verschiebung in der Lage ist.

9. Arzneimittelabgabevorrichtung nach Anspruch 8, wobei das hervorstehende Element (10') einen Kupplungskopf umfasst, und wobei der Verschiebungsteil ein drehbares Segment (21) des Kupplungskopfes umfasst.

10. Arzneimittelabgabevorrichtung nach Anspruch 8 oder 9, wobei das ausgesparte Mittel (12") einen Einsatz (24) umfasst und wobei der Verschiebungsteil einen Flansch (25) des Einsatzes (24) umfasst, wobei der Flansch (25) derart radial ablenkbar ist, dass ein Eintritt des hervorstehenden Elements (10, 10', 110, 210, 310, 410, 510) in das ausgesparte Mittel (12") ermöglicht und ein anschließender Austritt des hervorstehenden Elements (10, 10', 110, 210, 310, 410, 510) aus dem ausgesparten Mittel (12") verhindert wird.

11. Arzneimittelabgabevorrichtung nach einem der vorangehenden Ansprüche, wobei die Kolbenstange ein erstes Teil (307 und ein zum Kuppeln an das erste Teil angepasstes zweites Teil (360) umfasst.

12. Kolbenanordnung (100, 200, 300, 600, 700, 800) zur Verwendung in einer Arzneimittelabgabevorrichtung nach einem der Ansprüche 1-11, wobei die Kolbenanordnung (100, 200, 300, 600, 700, 800) umfasst:
- den hinteren Kolben (106, 206, 306, 606, 706, 806), der einen Körper, ein erstes Ende und ein zweites Ende umfasst, wobei der hintere Kolben (106, 206, 306, 606, 706, 806) ein Loch (140, 240, 340) aufweist, das sich durch den Körper vom ersten Ende zum zweiten Ende erstreckt,
- die Kolbenstange (107, 207, 307, 607, 707, 807), die derart angeordnet ist, dass sie den hinteren Kolben (106, 206, 306, 606, 706, 806) durchquert und aus dem ersten Ende hervorsteht,
- eine erste Kontaktfläche zum Inkontakttreten mit mindestens einem Teil des ersten Endes,
- eine zweite Kontaktfläche zum Inkontakttreten mit mindestens einem Teil des zweiten Endes und
- ein Mittel zum Variieren des Abstands zwischen der ersten Kontaktfläche und der zweiten Kontaktfläche.

13. Kolbenanordnung nach Anspruch 12, wobei das Mittel zum Variieren des Abstands zwischen der ersten Kontaktfläche und der zweiten Kontaktfläche eine Schraubengewindeverbindung zwischen der Kolbenstange (107, 207, 607, 707, 807) und ein damit verbundenes Element (138, 238, 638, 738, 838) umfasst.

14. Kolbenanordnung nach Anspruch 12, wobei das Mittel zum Variieren des Abstands zwischen der ersten Kontaktfläche und der zweiten Kontaktfläche eine Schraubengewindeverbindung zwischen einem ersten Teil (339) und einem zweiten Teil (361) der Kolbenstange (307) umfasst.

15. Verfahren zum Kuppeln eines ersten Kolbens und eines zweiten Kolbens in einer medizinischen Vorrichtung, wobei der erste Kolben a) ein erstes Material mit einem ersten Starrheitsmodul und b) ein erstes Kupplungsmittel umfasst, und der zweite Kolben ein zweites Material mit einem zweiten Starrheitsmodul umfasst, wobei der zweite Kolben des Weiteren mit einem zweiten Kupplungsmittel verbunden ist, wobei mindestens eines des ersten Kupplungsmittels und des zweiten Kupplungsmittels ein Material mit einem Starrheitsmodul umfasst, das höher als das erste und/oder zweite Starrheitsmodul ist, wobei das Verfahren umfasst
o Bewegen des zweiten Kolbens zum ersten Kolben, bis das erste Kupplungsmittel und das zweite Kupplungsmittel in einander eingreifen.

## Revendications

1. Un dispositif de délivrance de médicament (1) comprenant :
- un conteneur (50) comprenant une première extrémité (16) et une seconde extrémité (3),
- un piston avant (5, 5") disposé entre la première extrémité (16) et la seconde extrémité (3), définissant entre celles-ci une première chambre à volume variable (17),
- un piston arrière (6, 106, 206, 306, 406, 506, 606, 706, 806) disposé entre le piston avant (5, 5") et la seconde extrémité (3), définissant une seconde chambre à volume variable (18) entre celles-ci, le piston arrière (6, 106, 206, 306, 406, 506, 606, 706, 806) comprenant un matériau présentant un premier module de rigidité,
- une tige de piston (7, 7', 107, 207, 307, 407, 507, 607, 707, 807) apte à déplacer le piston arrière (6, 106, 206, 306, 406, 506, 606, 706, 806) par rapport au conteneur (50),
- une première substance placée dans la première chambre à volume variable (17),
- une seconde substance placée dans la seconde chambre à volume variable (18),
- des moyens de passage (15) aptes à permettre à la seconde substance de se déplacer de la seconde chambre à volume variable (18) vers la première chambre à volume variable (17), et
- des moyens de couplage structurés pour coupler de manière non libérable le piston avant (5, 5") et le piston arrière (6, 106, 206, 306, 406, 506, 606, 706, 806), les moyens de couplage comprenant un matériau présentant un second module de rigidité,
dans lequel le second module de rigidité est supérieur au premier module de rigidité.

2. Un dispositif de délivrance de médicament selon la revendication 1, dans lequel les moyens de couplage comprennent un organe saillant (10, 10', 110, 210, 310, 410, 510) et des moyens en évidement (12, 12") aptes à recevoir l'organe saillant (10, 10', 110, 210, 310, 410, 510), et dans lequel au moins une partie de l'organe saillant (10) ou au moins une partie des moyens en évidement (12, 12") est du matériau présentant le second module de rigidité.

3. Un dispositif de délivrance de médicament selon la revendication 2, dans lequel l'organe saillant (10, 10', 110, 210, 310, 410, 510) est associé à l'un d'entre le piston avant (5, 5"), le piston arrière (6, 106, 206, 306, 406, 506, 606, 706, 806) et la tige de piston (7, 7', 107, 207, 307, 407, 507, 607, 707, 807).

4. Un dispositif de délivrance de médicament selon la revendication 3, dans lequel l'organe saillant (10, 10', 110, 210, 310, 410, 510) est une partie intégrante de la tige de piston (7, 7', 107, 207, 307, 407, 507, 607, 707, 807).

5. Un dispositif de délivrance de médicament selon l'une des revendications 2 à 4, dans lequel les moyens en évidement (12") comprennent un insert (24), l'insert (24) comprenant un matériau présentant le second module de rigidité.

6. Un dispositif de délivrance de médicament selon l'une des revendications 2 à 5, dans lequel l'organe saillant (10, 10', 110, 210, 310, 410, 510) ou les moyens en évidement (12, 12") comprennent des moyens d'évacuation de fluide permettant à du fluide de passer l'organe saillant (10) pendant la connexion aux moyens en évidement (12, 12").

7. Un dispositif de délivrance de médicament selon la revendication 6, dans lequel l'organe saillant (10, 10') comprend une tête de couplage, et dans lequel les moyens d'évacuation de fluide comprennent une fente (20, 20') dans la tête de couplage.

8. Un dispositif de délivrance de médicament selon l'une des revendications 2 à 7, dans lequel l'organe saillant (10, 10', 110, 210, 310, 410, 510) et/ou les moyens en évidement (12, 12") comprennent une partie de déplacement qui est capable d'un déplacement radial.

9. Un dispositif de délivrance de médicament selon la revendication 8, dans lequel l'organe saillant (10') comprend une tête de couplage, et dans lequel la partie de déplacement comprend un segment pivotant (21) de la tête de couplage.

10. Un dispositif de délivrance de médicament selon la revendication 8 ou 9, dans lequel les moyens en évidement (12") comprennent un insert (24), et dans lequel la partie de déplacement comprend un flasque (25) de l'insert (24), le flasque (25) pouvant être fléchi radialement de manière à permettre l'entrée de l'organe saillant (10, 10', 110, 210, 310, 410, 510) dans les moyens en évidement (12") et à empêcher un échappement ultérieur de l'organe saillant (10, 10', 110, 210, 310, 410, 510) d'avec les moyens en évidement (12").

11. Un dispositif de délivrance de médicament selon l'une des revendications précédentes, dans lequel la tige de piston comprend une première partie (307) et une seconde partie (360) apte à être couplée à la première profondeur.

12. Un ensemble à piston (100, 200, 300, 600, 700, 800) pour une utilisation dans un dispositif de délivrance de médicament selon l'une des revendications 1 à 11, l'ensemble à piston (100, 200, 300, 600, 700, 800) comprenant :
- le piston arrière (106, 206, 306, 606, 706, 806) comprenant un corps, une première extrémité et une seconde extrémité, le piston arrière (106, 206, 306, 606, 706, 806) possédant un orifice (140, 240, 340) s'étendant au travers du corps depuis la première extrémité jusqu'à la seconde extrémité,
- la tige de piston (107, 207, 307, 607, 707, 807) configurée de manière à traverser le piston arrière (106, 206, 306, 606, 706, 806) et faire saillie à partir de la première extrémité,
- une première face de contact pour venir en contact avec au moins une partie de la première extrémité,
- une seconde face de contact pour venir en contact avec au moins une partie de la seconde extrémité, et
- des moyens pour faire varier la distance entre la première face de contact et la seconde face de contact.

13. Un ensemble à piston selon la revendication 12, dans lequel les moyens pour faire varier la distance entre la première face de contact et la seconde face de contact comprennent une liaison à filetage de vis entre la tige de piston (107, 207, 607, 707, 807) et un élément associé (138, 238, 638, 738, 838).

14. Un ensemble à piston selon la revendication 12, dans lequel les moyens pour faire varier la distance entre la première face de contact et la seconde face de contact comprennent une liaison à filetage à vis entre une première partie (339) et une seconde partie (361) de la tige de piston (307).

15. Un procédé pour coupler un premier piston et un second piston dans un dispositif médical, le premier piston comprenant a) un premier matériau présentant un premier module de rigidité et b) des premiers moyens de couplage, et le second piston comprenant un second matériau présentant un second module de rigidité, le second piston étant en outre associé à des seconds moyens de couplage, au moins l'un des premiers moyens de couplage et des seconds moyens de couplage comprenant un matériau présentant un module de rigidité qui est supérieur au premier et/ou au second module de rigidité, le procédé comprenant :
■ le déplacement du second piston en direction du premier piston jusqu'à ce que les premiers moyens de couplage et les seconds moyens de couplage se verrouillent réciproquement.
